# DEMANDE DE BREVET EUROPEEN

(11) **EP 0 864 321 A1**
(43) Date de publication de la demande: **16.09.1998**
(21) Numéro de dépôt: 98400489.5
(22) Date de dépôt: 02.03.1998
(51) Int. Cl.: A61K 7/48, A61L 27/00

(54) **Composition à base de collagène, son procédé de préparation et ses applications**

(30) Priorité: 13.03.1997 FR 9703006
(71) Demandeur: Laboratoire Cosmétique de Lécousse, 75002 Paris (FR)
(72) Inventeur: Motte, Dominique, 1008 Jouxtens-Mezery (FR); Volle, Isabelle, 69003 Lyon (CH)
(74) Mandataire: Eidelsberg, Victor Albert

(57) **Abrégé**

Procédé de préparation d'une composition à base de collagène consistant à broyer des fibres de collagène à une granulométrie comprise entre 10 et 100 microns et à les mélanger à de l'eau en une quantité suffisante pour donner un gel.

## Description

Dans le but de contribuer au soin de la peau, les formulations contenant des collagènes de différents types et extraits de l'animal, de mammifères ou de poissons, sont nombreuses.

On trouve dans l'arsenal de la cosmétique des produits de soin, de beauté, d'hygiène sous formes liquide, pâteuse, contenant du collagène.

On trouve aussi des masques ou des compresses de collagène lyophilisées, lesquels sont destinés à des usages dermiques pour des applications aussi bien thérapeutiques que cosmétiques.

On trouve aussi des formes plus élaborées comme des films, des sphérules, des poudres, contenant du collagène pour les domaines plus médicaux.

Les procédés d'obtention du collagène sont largement connus, ainsi à partir de dermes animaux, qui sont broyés, ensuite traités en solution acide, on extrait la fraction soluble du collagène. Dans certains cas, il est utile d'obtenir des fibres. A partir de ces fibres que l'on gélifie, on obtient des masques par lyophilisation. De même en réticulant la fraction soluble du collagène, on peut aussi par lyophilisation obtenir des films, des masques ou des compresses.

EP 0 709 101 décrit la préparation d'un gel thermostable à base de collagène nécessitant une dissolution à 50°C et sous agitation très rapide (turboagitateur). Ce gel est une préparation industrielle vendue en l'état.

Toutes ces formes de composition à base de collagène nécessitent pour leur fabrication une lyophilisation et sont donc onéreuses.

L'invention pallie ces inconvénients par un procédé de préparation d'une composition à base de collagène qui est bien plus simple à mettre en oeuvre qu'auparavant et,en conséquence,bien moins coûteuse.

Le procédé consiste à broyer des fibres de collagène à une granulométrie compris entre 10 et 100 microns et à les mélanger à de l'eau et, éventuellement à un polysaccharide, en une quantité suffisante pour donner un gel. On obtient ainsi une composition à base de collagène sous la forme d'un gel qui comprend de 1 à 2 parties en poids de collagène et, de manière correspondante, de 99 à 98 parties d'eau.

D'une manière inattendue, il s'est avéré que, lorsque l'on met des fibres de collagène, broyées à la granulométrie indiquée, en contact avec de l'eau, il se forme, notamment à la température ambiante (4 à 28°C) par simple agitation manuelle en moins de 15 minutes, un gel ayant une viscosité suffisante, notamment comprise entre 8000 et 80000 cps, telle que mesurée à la température ambiante au viscosimètre Brookfield module 5, pour permettre immédiatement la formation d'un masque à base de collagène utile en cosmétique ou en thérapeutique.

De préférence, on ajoute à la composition de 0,1 à 0,8 partie en poids de polysaccharide qui peuvent être, de préférence, des gommes, telle que la gomme de guar et la gomme de xanthane.

Le produit est vendu de préférence en deux pots, l'un contenant les fibres de collagène éventuellement additionnées de polysaccharide, l'autre de l'eau. L'utilisateur mélange le contenu des deux pots au dernier moment, lorsqu'il veut préparer le masque en sorte que le gel préparé est frais et que ses constituants ne se séparent pas au cours du temps.

Le premier stade du procédé consiste à broyer des fibres de collagène animales, de mammifères ou de poissons à la granulométrie requise. Les fibres utilisées peuvent être obtenues comme il est connu, en traitant les matières brutes à la soude ou en présence d'enzymes comme la pepsine. Pour les réduire à la granulométrie requise, on peut les broyer à l'aide de broyeur à couteaux. On peut aussi utiliser la technique de cryobroyage, avec par exemple de l'azote liquide. Ce stade peut s'effectuer en usine.

Le deuxième stade du procédé, qui peut s'effectuer extemporanément chez l'utilisateur, consiste à ajouter à cette poudre de l'eau. On peut si on le souhaite, avant d'ajouter l'eau, mélanger à la poudre fine des polysaccharides qui participeront à la gélification rapide et homogène de la poudre. Les quantités de polysaccharides utilisées seront comprises entre 10 et 40% du poids de fibres mis en oeuvre, avec une préférence pour 20%, étant entendu que le mieux est que ces polysaccharides soient ajoutées en usine aux fibres de collagène.

Il pourra être incorporé à la poudre finale des principes actifs qui seront libérés sur la peau lors de leur réhydratation, au cours du soin. Ces principes actifs hydrosolubles sont choisis parmi les vitamines solubles, les oligo-éléments, des extraits végétaux comme par exemple les saponines. Ces additions pourront varier en fonction de l'objectif recherché par le formulateur ou le préparateur.

Suivant les principes actifs, la composition peut être protectrice en maintenant les fonctions normales de la peau ou en les optimisant par hydratation par exemple. Elle peut soigner les altérations d'ordre esthétique comme les rougeurs ou dermatologie comme notamment l'acnée, érythème, hématome.

Les exemples suivants illustrent l'invention :

### Exemple 1

### Obtention de 100 grammes de poudre gélifiable.

On part d'un kilogramme de derme de veau d'âge inférieur à 6 mois.

Le derme est transformé par les moyens connus, en fibres soit par traitement préalable à la soude, suivi d'une précipitation au chlorure de sodium à pH acide, dessalage et séchage à l'acétone du précipité par répétition des bains.

On obtient quelques 85 grammes de fibres séchées, que l'on cryobroie sous azote liquide directement introduite dans le broyeur.

Un litre d'azote est nécessaire pour cette opération.

La poudre présente une granulométrie moyenne de 50 µm.

On obtient 80 grammes de poudre de fibres finement broyées, auxquels on mélange 20 grammes de gomme de marque Jaguar de la société Rhone-Poulenc à base de gomme de guar.

Un gramme de cette poudre est gélifiée en lui rajoutant 99 ml d'eau.

Cette préparation en forme de gel permet en cabine de recouvrir le visage ou la partie du corps à traiter.

### Exemple 2

Obtention comme à l'exemple 1, de poudre de collagène de poisson ou collagène marin. Celui-ci est obtenu à partir de peau de soles, poissons d'eau chaude, offrant ainsi une meilleure résistance thermique, par broyage des peaux de sole, lavages par solution de tampon au phosphate, traitement enzymatique à la pepsine, précipitation au NaCl à pH inférieur à 5, lavage successif avec un mélange eau/acétone 50/50, séchage à l'étuve et cryobroyage comme à l'exemple 1. Le rendement est de 10 %. 1 à 2 g de cette poudre est gélifié par 99 à 98 ml d'eau.

### Exemple 3

Masques pelables, ils sont confectionnés en rajoutant à la poudre de l'exemple 1 2g de fibres de cellulose.

### Exemple 4

Dans le gel obtenu dans l'exemple 1 ou 2, on rajoute des saponines de ficaire, à raison de 2% en poids de gel. Le produit obtenu est destiné à lutter contre les agressions solaires sur la peau.

### Exemple 5

Avec la même technique initiale qu'à l'exemple 4, mais avec 5% d'un nébulisat d'arnica, à la place des saponines, on obtient d'un gel visant à décongestionner un hématome.

### Exemple 6

La poudre est gélifiée avec un extrait aqueux d'Aloe à 5%, cette préparation est utilisée en vue de la réhydratation cutanée.

### Exemple 7

Conception d'une formule "anti age" :

On complète la formulation de base de l'exemple 1 ou 9 avec des extraits actifs :
2% d'élastine marine
0,08% d'acide hyaluronique
0,5% de vitamines hydrosolubles
2% de ginko biloba

### Exemple 8

Conception d'une formule "rougeurs diffuses" :

On complète la formulation de base de l'exemple 1 ou 9 avec des extraits végétaux
3% d'extrait de Centella Asiatica
2% d'extrait de Fragon
1,5% d'extrait de Hydrastis

### Exemple 9

On reprend l'exemple 1, mais sans addition de gomme. On obtient un gel qui permet de recouvrir le visage.

## Revendications

1. Procédé de préparation d'une composition à base de collagène consistant à broyer des fibres de collagène à une granulométrie comprise entre 10 et 100 microns et à les mélanger à de l'eau en une quantité suffisante pour donner un gel.

2. Procédé suivant la revendication 1, caractérisé en ce qu'il consiste à mélanger de 1 à 2 parties en poids de fibres de collagène d'une granulométrie comprise entre 10 et 100 microns et de 99 à 98 parties d'eau.

3. Procédé suivant la revendication 2, caractérisé en ce qu'il consiste à mélanger de 0,1 à 0,8 parties en poids d'un polysaccharide, tel que de la gomme de guar ou de la gomme de xanthane, aux fibres de collagène.

4. Composition à base de collagène, caractérisée en ce qu'elle comprend des fibres de collagène d'une granulométrie comprise entre 10 et 100 microns.

5. Composition suivant la revendication 4, caractérisée en ce qu'elle comprend un polysaccharide représentant de 10 à 40 % du poids des fibres de collagène.

6. Nécessaire pour préparer un gel, caractérisé en ce qu'il comprend dans un premier pot de 99 à 98 parties en poids d'eau et dans un deuxième pot de 1 à 2 parties en poids de fibres de collagène d'une granulométrie comprise entre 10 et 100 microns.

7. Nécessaire pour préparer un gel suivant la revendication 6, caractérisé en ce que le deuxième pot comprend de 0,1 à 0,8 parties en poids d'un polysaccharide.

8. L'utilisation d'une composition suivant l'une des revendications 1 à 4, 6 ou 7, en cosmétique ou en thérapeutique, notamment pour préparer un masque.
